# EUROPEAN PATENT APPLICATION

(11) **EP 3 103 444 A1**
(43) Date of publication of application: **14.12.2016**
(21) Application number: 16173461.1
(22) Date of filing: 08.06.2016
(51) Int. Cl.: A61K 9/20, A61K 9/24, A61K 31/165, A61K 31/55

(54) **PHARMACEUTICAL COMPOSITIONS OF LACOSAMIDE AND ESLICARBAZEPINE**

(30) Priority: 09.06.2015 TR 201507051
(71) Applicant: Sanovel Ilac Sanayi ve Ticaret A.S., 34460 Istanbul (TR)
(72) Inventor: TÜRKYILMAZ, Ali, 34460 Istanbul (TR); YELKEN, Gülay, 34460 Istanbul (TR); HATIRNAZ, Zekiye Basak, 34460 Istanbul (TR); ECEOGLU, Melike, 34460 Istanbul (TR)
(74) Representative: Sevinç, Erkan

(57) **Abstract**

The present invention relates to a pharmaceutical composition comprising lacosamide or a pharmaceutically acceptable salt in combination with eslicarbazepine or a pharmaceutically acceptable salt thereof and at least one pharmaceutically acceptable excipient.

## Description

### Field of Invention

The present invention relates to a pharmaceutical composition comprising **lacosamide** or a pharmaceutically acceptable salt in combination with **eslicarbazepine** or a pharmaceutically acceptable salt thereof and at least one pharmaceutically acceptable excipient.

### Background of Invention

Lacosamide is an aminoacid derivative with an anticonvulsant activity useful in the adjunctive treatment of partial-onset seizures with or without secondary generalization in adults with epilepsy. It is also called as erlosamide or harkoseride. Its chemical name is (2R)-2-(Acetylamino)-3-methoxy-N-(phenylmethyl) propanamide and its chemical structure is shown in the Formula 1.

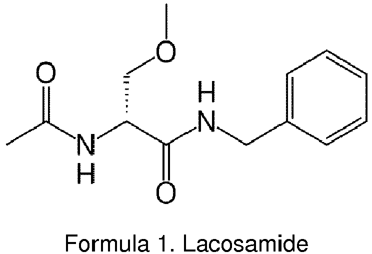

Lacosamide is sparingly soluble in water and it has low flowability, which makes the tableting process difficult at the high amount of active substance in tablets.

There is lacosamide immediate release formulation licensed in the US and Europe in the form of immediate release tablets, oral solutions and intravenous injection solutions under the brand name Vimpat^{®} by UCB. Vimpat® coated IR tablet has crospovidone as disintegrant agent, microcrystalline cellulose, HPC (low substituted) and HPC as filler and binder, silicified microcrystalline cellulose as glidant, colloidal silicon dioxide as filler, magnesium stearate as lubricant and a non-functional coating. It may be taken with or without food. The initial dose should be 50 mg twice daily (100 mg per day). Vimpat® IR tablet is prepared by wet granulation. To overcome poor flowability of lacosamide, wet granulation comprising HPC (including low substituted HPC) is selected.

Lacosamide can be increased at weekly intervals by 100 mg/day given as two divided doses up to the recommended maintenance dose of 200 to 400 mg/day, based on individual patient response and tolerability. The currently commercially available tablets are available in strengths of 50, 100, 150 and 200 mg of lacosamide.

Eslicarbazepine acetate is a molecule blocking voltage-gated sodium channels, providing anticonvulsant effect, and used in treating epilepsy and neuropathic pain. Its chemical designation is (S)-10-acetoxy-10,11-dihydro-5H-dibenzo[b,f]azepine-5-carboxamide, with the chemical structure illustrated below in Formula 2.

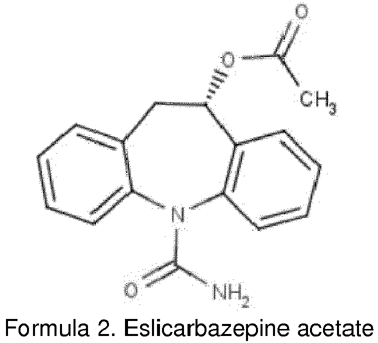

Eslicarbazepine acetate has an optically-active chiral center. It is structurally analogous of carbamazepine and oxcarbazepine, and does not contain any toxic metabolites and unwanted enantiomeric impurities.

Various formulations have been developed in relation to eslicarbazepine acetate. There are conventional tablet formulations available, containing the eslicarbazepine acetate molecule. It is commercially-available under the tradename Aptiom^{®} in 200, 400, 600, and 800 mg tablets.

Various applications are also available in the patent literature in relation to eslicarbazepine. In the patent application WO2009054743 is disclosed a pharmaceutical formulation comprising licarbazepine acetate, a binder, and a disintegrant. According to that publication, at least part of the disintegrant is present in the granules (intragranular), and at least part of the disintegrant is extragranular.

There are several types of antiepileptic drugs (AEDs), which reduce the frequency and/or severity of seizures and many of which may work differently and may have different indications and usage. The precise mechanisms of antiepileptic effects of lacosamide and eslicarbazepine in humans remains to be fully elucidated. Lacosamide used as monotherapy or add-on therapy, it selectively enhances slow inactivation of voltage-gated sodium channels. On the other side, eslicarbazepine is thought to involve inhibition of voltage-gated sodium channels. Studies indicate that eslicarbazepine stabilizes the voltage-gated sodium channel at a certain point during the nerve impulse. This inhibits repetitive neuronal firing and may result in reduced seizure activity. The aim of this present invention is to combine these two antiepileptic drugs in the same dosage form.

Conventional epilepsy therapy generally involves administration of one or more different epilepsy drugs. Different drugs in one composition is preferred for the patient compliance and ease of use. However, not all compositions comprising more than one epilepsy drugs are more suitable, in terms of safety or efficacy, than the administration of a single drug.

It is well known that drugs used in the same therapeutic area or even for treating the same indication cannot always be combined *a priori* with the expectation of at least additive therapeutic effects. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, unwanted drug interactions or additional side effects. More specifically, in the area of epilepsy there are drug combinations that are contraindicated for some or all of these very same reasons.

Thus, there is a need in the art for a pharmaceutical composition or a dosage form comprising a combination of lacosamide and eslicarbazepine to achieve improved epilepsy treatment. In this present invention, a pharmaceutical composition comprising lacosamide and eslicarbazepine has been developed.

### Description of the invention

The present invention relates to a pharmaceutical composition comprising **lacosamide** or a pharmaceutically acceptable salt in combination with **eslicarbazepine** or a pharmaceutically acceptable salt thereof and at least one pharmaceutically acceptable excipient.

One embodiment of this present invention is to provide improved epilepsy treatment that reduces repetitive neuronal firing and seizure activity with a pharmaceutical composition comprising lacosamide or a pharmaceutically acceptable salt in combination with **eslicarbazepine** or a pharmaceutically acceptable salt thereof which overcomes the above described problems in prior art and have additional advantages over them.

According to one embodiment, said pharmaceutically acceptable salt of eslicarbazepine is eslicarbazepine acetate.

According to this embodiment, eslicarbazepine acetate is present in an amount of between 5.0 to 98.0 %, preferably between 5.0 to 85.0 % and more preferably it is 15.0 to 85.0 % by weight of total formulation.

According to this embodiment, eslicarbazepine acetate is present in an amount of between 50 to 1200 mg, preferably 100 to 800 mg and more preferably it is 200 to 800 mg.

In one embodiment, lacosamide is present in an amount of between 5.0 to 98.0 %, preferably between 5.0 to 85.0 % and more preferably it is 5.0 to 70.0 % by weight of total formulation.

According to this embodiment, lacosamide is present in an amount of between 50 to 800 mg, preferably 100 to 800 mg and more preferably it is 100 to 400 mg.

Using different drugs may induce incompatibility problems and undesired dissolution profiles that cause undesired side effects. In this present invention, a pharmaceutical dosage form comprising lacosamide in combination with eslicarbazepine acetate has been developed with safe and effective dissolution profiles for each drug molecule.

In one embodiment, in this present invention, pharmaceutical combination formulated in one dosage form provides desired dissolution profile for both lacosamide and eslicarbazepine.

According to this embodiment, the ratio of lacosamide to eslicarbazepine acetate is between 0.05 - 30.00 (w/w), preferably 0.15 - 20.0 (w/w) and more preferably 0.20 - 15.0 (w/w).

According to one embodiment, the ratios used in this present invention ensure the required effective doses for the treatment and desired dissolution profile for both lacosamide and eslicarbazepine acetate.

An embodiment of this present invention is to combine lacosamide and eslicarbazepine in a same and stable dosage form. Pharmaceutical composition of this present invention comprising lacosamide in combination with eslicarbazepine acetate is in the form of a coated tablet, tablet, bilayer tablet, multilayer tablet, capsule, tablet in tablet, an inlay tablet, injectable preparate, suspension, syrup, sachet, ointment, cream or gel.

In one embodiment, pharmaceutical composition of this present invention comprising lacosamide in combination with eslicarbazepine acetate is in the form of a coated tablet, tablet or bilayer tablet.

In this embodiment, pharmaceutical composition of this present invention is in the form of a coated tablet.

In another embodiment, to ensure the stability of both lacosamide and eslicarbazepine acetate in the same dosage form, in this present invention, coating is used to protect the pharmaceutical composition from the moisture.

According to this embodiment, pharmaceutical composition of this present invention is in the form of a coated tablet. To provide coated tablets, suitable coating agent that is not chemically interact with both lacosamide and eslicarbazepine acetate is used.

In this embodiment, coating agent is selected from the group comprising polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinyl alcohol based coating agents, aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene oxide, poly-DL-(lactic acid), polyvinyl acetate phthalate, potassium chloride, shellac, shellac with stearic acid, sucrose, surface color agents, titanium oxide, tributyl citrate, triethyl citrate, vanillin, white wax, xylitol, yellow wax, zein, dimethylaminoethyl methacrylate, butyl methacrylate and methyl methacrylate (Eudragit E 100) (Poly(butyl methacrylate-co-(2-demethylaminoeethyl)methacrylate-co-methyl methacrylate)) or hydroxypropyl methyl cellulose (HPMC), polyethyleneglycol (PEG), polivinylpyrrolidon (PVP), polyvinyl alcohol (PVA), vinylpyrrolidone-vinyl acetate copolymer (PVP- PVAc) and all kinds of Opadry derivatives such as Opadry pink II, as well as pigments, dyes, titanium dioxide and iron oxide, polymethylmetacrylate copolymers (Eudragit), titanium dioxide, dyes and iron oxide and talc or mixtures thereof.
In this embodiment, coating agent comprises polyvinyl alcohol-polyethylene glycol copolymer.

Besides the moisture sensitivity problem, having amine group is also another stability problem for eslicarbazepine acetate and lacosamide. They may react with many excipients or impurities of excipients. For example, it is known by the skilled person in the art that primer and seconder amine group containing compounds such as eslicarbazepine acetate and lacosamide may give Maillard reaction with lactose. This may give impurities which cause incompatibility or undesired dissolution profile for both lacosamide and eslicarbazepine acetate in the composition.

According to the challenges in terms of combining lacosamide and eslicarbazepine in one stable dosage form, the selection of the excipients is essential. According to further embodiment, at least one pharmaceutically acceptable excipient is selected from the group comprising lubricants, glidants, disintegrants, buffering agents, stabilizers, binders, diluents, dispersing agents, plasticizers, preservatives, sweeteners, flavoring agents, coloring agents, coating agents or mixtures thereof.

In another embodiment, due to flowability problems of lacosamide, during the production of pharmaceutical composition comprising lacosamide and eslicarbazepine acetate, selection of lubricant is important. According to this embodiment, the pharmaceutical formulation comprises a lubricant selected from the group comprising sodium stearyl fumarate, polyethylene glycol, sodium benzoate, magnesium stearate, calcium stearate or a mixture thereof, preferably sodium stearyl fumarate. Hydrophilic properties of sodium stearyl fumarate cause a decrease in process time with mixing short time. Moreover, it is surprisingly found that desired dissolution profile is also achieved with sodium stearyl fumarate.

The amount of sodium stearyl fumarate is present in an amount of 0.01 to 10.00 %, preferably 0.10 to 5.00 %, more preferably it is 0.25 to 2.00 % by weight of total composition.

Suitable glidants may include but not limited to colloidal silicon dioxide, talc, aluminium silicate and the like and mixtures thereof.

Suitable binders may include but not limited to polyvinylpyrrolidone, microcrystalline cellulose, polyethylene glycol, polyvinyl alcohol, polyvinyl acetate and their copolymers, cellulose derivatives such as hydroxypropyl methyl cellulose, carboxy methyl cellulose, methyl cellulose, gelatin, polyvinylalcohol, carrageenan, guar gum and the like and mixtures thereof.

Suitable disintegrants may comprise but not limited to starch, crospovidone (cross-linked polyvinil pyrrolidone), povidone, poloxomer, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof.

Suitable buffering agents may comprise but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate or mixtures thereof.

Suitable stabilizers may comprise but not limited to citric acid, fumaric acid, tartaric acid, sodium citrate, sodium benzoate, sodium dihydrogen phosphate, calcium carbonate, magnesium carbonate, arginine, lysine, meglamine, tocopherol, butylhydroxyanisole (BHA), butylhydroxytoluene (BHT), ascorbic acid, gallic acid esters or the mixtures thereof, and preferably, citric acid, fumaric acid, arginine or mixtures thereof.

Suitable binders may include but not limited to copovidone, polymethyl methacrylate derivatives, hydroxypropyl methyl cellulose, polyvinylpyrrolidone, polyethylene glycol, polyvinyl alcohol, starch, pregelatinized starch, glucose, glucose syrup, natural gums, sucrose, sodium alginate, hydroxypropyl cellulose, carboxy methyl cellulose, methyl cellulose, gelatin, carrageenan, guar gum, carbomer, polymethacrylates, methacrylate polymers, collagens gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable diluents may comprise but not limited to lactose monohydrate, lactose, dibasic calcium phosphate, microcrystalline cellulose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

Suitable dispersing agents may comprise but not limited to calcium silicate, magnesium aluminum silicate or mixtures thereof.

Suitable plasticizers may comprise but not limited to polyethylene glycols of different molecular weights, propylene glycol, glycerine, triethyl citrate (TEC), triacetin, diethyl phthalate (DEP), dibutyl phthalate (DBP), tributhyl citrate (TBC), castor oil, dibutyl sebacate (DBS), diacetylated monogglycerides or mixtures thereof.

Suitable preservatives may comprise but not limited to methyl paraben, propyl paraben and their salts (such as sodium, potassium), sodium benzoate, citric acid, benzoic acid, butylated hydroxytoluene, boric acid, sorbic acid, benzyl alcohol, benzalconium chloride, parahydroxybenzoic acids or butylated hydroxyanisole or mixtures thereof.

Suitable sweeteners may comprise but not limited to aspartame, potassium acesulfame, sodium saccharinate, neohesperidine dihydrochalcone, sucralose, saccharin, sugars such as sucrose, glucose, lactose, fructose or sugar alcohols such as mannitol, sorbitol, xylitol, erythritol or mixtures thereof.

Suitable flavoring agents may comprise but not limited to menthol, peppermint, cinnamon, chocolate, vanillin or fruit essences such as cherry, orange, strawberry, grape, black currant, raspberry, banana, red fruits, wild berries or mixtures thereof.

Suitable coloring agents may comprise but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

### Example 1: Wet granulation - coated tablet

| **ingredients** | **amount (%)** |
|---|---|
| lacosamide | 5.00-70.00 |
| mannitol | 5.00-90.00 |
| dibasic calcium phosphate | 5.00-90.00 |
| crospovidone | 1.00-30.00 |
| polyvinylpyrrolidone | 0.10-25.00 |
| eslicarbazepine acetate | 5.00-85.00 |
| microcrystalline cellulose | 5.00-90.00 |
| polyvinylpyrrolidone | 0.10-25.00 |
| | |
| silicon dioxide | 0.10-0.20 |
| magnesium stearate | 0.25-2.00 |
| Kollicoat IR | 0.25-3.00 |

Production process Lacosamide, crospovidon, dibasic calcium phosphate and mannitol are mixed. Granulation is done with alcoholic/hydroalcoholic polyvinylpyrrolidone solution Granules are dried and sieved. Eslicarbazepine acetate and microcrystalline cellulose are mixed and granulated with alcoholic/hydroalcoholic polyvinylpyrrolidone solution. Then, dried and sieved. Lacosamide granules and eslicarbazepine acetate granules are first mixed silicon dioxide. Then mixed with magnesium stearate. Tablet compression is performed. Coating is performed with Kollicoat IR.

### Example 2: Direct compression - coated tablet

| **ingredients** | **amount (%)** |
|---|---|
| lacosamide | 5.00-70.00 |
| Eslicarbazepine acetate | 5.00-85.00 |
| starch | 5.00-90.00 |
| dibasic calcium phosphate | 5.00-90.00 |
| crospovidone | 1.00-30.00 |
| copovidone | 0.10-25.00 |
| silicon dioxide | 0.10-0.20 |
| sodium stearyl fumarate | 0.25-2.00 |
| Kollicoat IR | 0.25-3.00 |

**Production process:** Lacosamide, eslicarbazepine acetate, crospovidon, copovidon, starch and dibasic calcium phosphate are mixed. The mixture is mixed with silicon dioxide, then with sodium stearyl fumarate. Tablet compression is performed and coated with Kollicoat IR.

### Example 3: Direct compression - Bilayer Tablet

| **ingredients** | **amount (%)** |
|---|---|
| **1^{st} layer** | |
| lacosamide | 5.00-70.00 |
| mannitol | 5.00-90.00 |
| crospovidone | 1.00-30.00 |
| silicon dioxide | 0.10-0.20 |
| sodium stearyl fumarate | 0.25-2.00 |

| **2^{nd} layer** | |
|---|---|
| eslicarbazepine acetate | 5.00-85.00 |
| microcrystalline cellulose | 5.00-90.00 |
| dibasic calcium phosphate | 5.00-90.00 |
| crospovidone | 0.10 - 30.00 |
| Silicon dioxide | 0.10-0.20 |
| sodium stearyl fumarate | 0.25-2.00 |
| Kollicoat IR | 0.25-3.00 |

**Production process:** Lacosamide, crospovidon, mannitol and sodium stearyl fumarate are mixed. Eslicarbazepine acetate, dibasic calcium phosphate, microcrystalline cellulose and crospovidone are mixed. Each mixture is first mixed with silicon dioxide, then sodium stearyl fumarate. Mixtures are compressed into bilayer tablet. Coating is performed with Kollicoat IR.

### Example 4: Hot melt extrusion - Bilayer tablet

| **ingredients** | **amount (%)** |
|---|---|
| **1^{st} layer** | |
| lacosamide | 5.00-70.00 |
| lactose monohydrate | 5.00-90.00 |
| crospovidone | 1.00-30.00 |
| polyvinylpyrrolidone | 0.10-0.25 |
| sodium stearyl fumarate | 0.25-2.00 |

| **2^{nd} layer** | |
|---|---|
| Eslicarbazepine acetate | 5.00-85.00 |
| poloxamer | 0.50-15.00 |
| silicon dioxide | 0.10-0.20 |
| sodium stearyl fumarate | 0.25-2.00 |
| Kollicoat IR | 0.25-3.00 |

**Production process:** Lacosamide, lactose monohydrate and crospovidon are mixed. Granulation is done with alcoholic/hydroalcoholic polyvinylpyrrolidone solution Granules are dried and sieved. Eslicarbazepine acetate and poloxamer passed through the extruder by melting. It is cooled and sieved. Each mixture is first mixed with silicon dioxide, then sodium stearyl fumarate. Mixtures are compressed into bilayer tablet. Coating is performed with Kollicoat IR.

## Claims

1. A pharmaceutical composition comprising lacosamide or a pharmaceutically acceptable salt in combination with eslicarbazepine or a pharmaceutically acceptable salt thereof and at least one pharmaceutically acceptable excipient.

2. The pharmaceutical composition according to claim 1, wherein a pharmaceutically acceptable salt of eslicarbazepine is eslicarbazepine acetate.

3. The pharmaceutical composition according to claim 2, eslicarbazepine acetate is present in an amount of between 5.0 to 98.0 %, preferably between 5.0 to 85.0 % and more preferably it is 15.0 to 85.0 % by weight of total formulation.

4. The pharmaceutical composition according to claim 2, eslicarbazepine acetate is present in an amount of between 50 to 1200 mg, preferably 100 to 800 mg and more preferably it is 200 to 800 mg.

5. The pharmaceutical composition according to claim 1, lacosamide is present in an amount of between 5.0 to 98.0 %, preferably between 5.0 to 85.0 % and more preferably it is 5.0 to 70.0 % by weight of total formulation.

6. The pharmaceutical composition according to claim 1, lacosamide is present in an amount of between 50 to 800 mg, preferably 100 to 800 mg and more preferably it is 100 to 400 mg.

7. The pharmaceutical composition according to claim 1 to 6, wherein the ratio of lacosamide to eslicarbazepine acetate is between 0.05 - 30.00 (w/w), preferably 0.15 - 20.0 (w/w) and more preferably 0.20 - 15.0 (w/w).

8. The pharmaceutical composition according to claim 1, wherein said pharmaceutical composition is in the form of a coated tablet, tablet, bilayer tablet, multilayer tablet, capsule, tablet in tablet, an inlay tablet, injectable preparat, suspension, syrup, sachet, ointment, cream or gel.

9. The pharmaceutical composition according to claim 1, wherein said pharmaceutical composition is in the form of a coated tablet, tablet or bilayer tablet.

10. The pharmaceutical composition according to claim 1, wherein said pharmaceutical composition is in the form of a coated tablet.

11. The pharmaceutical composition according to claim 10, wherein the coating agent is selected from the group comprising polyvinyl alcohol-polyethylene glycol copolymers, polyvinyl alcohol or copolymers, ethylcellulose dispersions, Kerry-HPC, polyethylene glycol, polyvinylprolidone, polyvinyl alcohol based coating agents, aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene oxide, poly-DL-(lactic acid), polyvinyl acetate phthalate, potassium chloride, shellac, shellac with stearic acid, sucrose, surface color agents, titanium oxide, tributyl citrate, triethyl citrate, vanillin, white wax, xylitol, yellow wax, zein, dimethylaminoethyl methacrylate, butyl methacrylate and methyl methacrylate (Poly(butyl methacrylate-co-(2-demethylaminoeethyl)methacrylate-co-methyl methacrylate)), hydroxypropyl methyl cellulose, polyethyleneglycol, polivinylpyrrolidon, polyvinyl alcohol, vinylpyrrolidone-vinyl acetate copolymer as well as pigments, dyes, titanium dioxide and iron oxide, polymethylmetacrylate copolymers (Eudragit), titanium dioxide, dyes and iron oxide and talc or mixtures thereof.

12. The pharmaceutical composition according to claim 11, wherein the coating agent comprises polyvinyl alcohol-polyethylene glycol copolymer.

13. The pharmaceutical composition according to claim 1, wherein at least one pharmaceutically acceptable excipient is selected from the group comprising lubricants, buffering agents, stabilizers, binders, diluents, dispersing agents, glidants, disintegrants, plasticizers, preservatives, sweeteners, flavoring agents, coloring agents, coating agents or mixtures thereof.

14. The pharmaceutical composition according to claim 13, wherein lubricant is selected from the group comprising sodium stearyl fumarate, polyethylene glycol, sodium benzoate, magnesium stearate, calcium stearate or a mixture thereof, preferably sodium stearyl fumarate.

15. The pharmaceutical composition according to any of the preceding claims, for use in the treatment of epilepsy and neuropathic pain.
